# EUROPÄISCHE PATENTSCHRIFT

(11) **EP 2 961 755 B1**
(45) Veröffentlichungstag und Bekanntmachung des Hinweises auf die Patenterteilung: **18.10.2017**
(21) Anmeldenummer: 14706592.4
(22) Anmeldetag: 25.02.2014
(51) Int. Cl.: C07D 519/00, A61K 31/519, A61P 9/10

(54) **TRIFLUORMETHYL-SUBSTITUIERTE ANNELLIERTE PYRIMIDINE UND IHRE VERWENDUNG**
TRIFLUOROMETHYL-SUBSTITUTED RING-FUSED PYRIMIDINES AND USE THEREOF
PYRIMIDINES ANNELÉES À SUBSITUTION TRIFLUOROMÉTHYLE ET UTILISATION CORRESPONDANTE

(30) Priorität: 01.03.2013 EP 13157434
(43) Veröffentlichungstag der Anmeldung: 06.01.2016
(73) Patentinhaber: Bayer Pharma Aktiengesellschaft, 13353 Berlin (DE)
(72) Erfinder: FOLLMANN, Markus, 50859 Köln (DE); STASCH, Johannes-Peter, 00046 Grottaferrata (RM) (IT); REDLICH, Gorden, 44799 Bochum (DE); LANG, Dieter, 42553 Velbert (DE); VAKALOPOULOS, Alexandros, 40721 Hilden (DE); WUNDER, Frank, 42117 Wuppertal (DE); TERSTEEGEN, Adrian, 42111 Wuppertal (DE)
(74) Vertreter: BIP Patents
(86) Internationale Anmeldenummer: PCT/EP2014/053636
(87) Internationale Veröffentlichungsnummer: WO 2014/131760

(56) Entgegenhaltungen:
- WO-A1-2013/004785
- WO-A1-2013/030288

## Beschreibung

Die vorliegende Anmeldung betrifft neue Trifluormethyl-substituierte annellierte Pyrimidine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

Vor einigen Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681]. Zu den neueren Stimulatoren der löslichen Guanylatzyklase gehören u.a. BAY 41-2272, BAY 41-8543 und Riociguat (BAY 63-2521) (siehe z.B. Stasch J.-P. et al., Nat. Rev. Drug Disc. 2006; 5: 755-768; Stasch J.-P. et al., ChemMedChem 2009; 4: 853-865. Stasch J.-P. et al., Circulation 2011; 123: 2263-2273).

Als Stimulatoren der löslichen Guanylatcyclase werden in WO 00/06568 und WO 00/06569 annellierte Pyrazol-Derivate und in WO 03/095451 Carbamat-substitutierte 3-Pyrimidinyl-Pyrazolopyridine offenbart. 3-Pyrimidinyl-Pyrazolopyridine mit Phenylamid-Substituenten werden in E. M. Becker et al., BMC Pharmacology 1 (13), 2001 beschrieben. WO 2004/009590 beschreibt Pyrazolopyridine mit substituierten 4-Aminopyrimidinen zur Behandlung von ZNS-Erkrankungen. WO 2010/065275 und WO 2011/149921 offenbaren substituierte Pyrrolo- und Dihydropyridopyrimidine als sGC Aktivatoren. Als sGC Stimulatoren werden in WO 2012/004259 annellierte Aminopyrimidine, in WO 2012/004258, WO 2013/004785 sowie WO 2013/030288 annellierte Pyrimidine und Triazine beschrieben. WO 2012/28647 offenbart Pyrazolopyridine mit verschiedenen Azaheterocyclen zur Behandlung kardiovaskulärer Erkrankungen.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung und/oder ihres Nebenwirkungsprofils.

Gegenstand der vorliegenden Erfindung ist die Verbindung mit dem systematischen Namen 2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on und der Strukturformel (I) 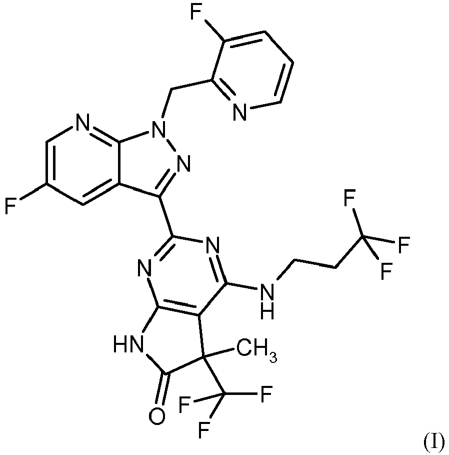 sowie ihre Salze, Solvate und Solvate der Salze.

Erfindungsgemäße Verbindungen sind die Verbindungen der Formel (I), (I-A) und (I-B) und deren Salze, Solvate und Solvate der Salze, die von Formel (I), (I-A) und (I-B) umfassten Verbindungen der nachfolgend genannten Formeln und deren Salze, Solvate und Solvate der Salze sowie die von Formel (I), (I-A) und (I-B) umfassten, nachfolgend als Ausführungsbeispiele genannten Verbindungen und deren Salze, Solvate und Solvate der Salze, soweit es sich bei den von Formel (I), (I-A) und (I-B) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in unterschiedlichen stereoisomeren Formen existieren, d.h. in Gestalt von Konfigurationsisomeren. Die vorliegende Erfindung umfasst deshalb die Enantiomere und ihre Mischungen. Aus solchen Mischungen von Enantiomeren lassen sich die stereoisomer einheitlichen Bestandteile in bekannter Weise isolieren; vorzugsweise werden hierfür chromatographische Verfahren verwendet, insbesondere die HPLC-Chromatographie an chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P ³³S, ³⁴S, ³⁵S, ³⁶S, ¹⁸F ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen. Bevorzugt ist im Rahmen der vorliegenden Erfindung das Enantiomer der Verbindung (I) mit dem systematischen Namen (5R)-2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on und der Strukturformel (I-A) 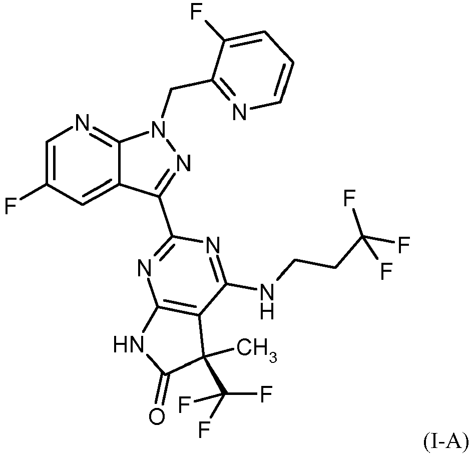 sowie seine Salze, Solvate und Solvate der Salze.

Bevorzugt ist im Rahmen der vorliegenden Erfindung das Enantiomer der Verbindung (I) mit dem systematischen Namen (5S)-2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on und der Strukturformel (I-B) 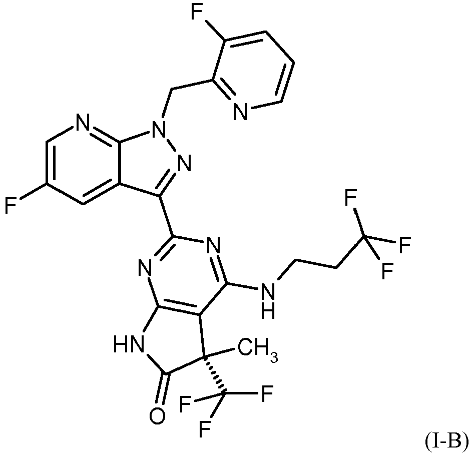 sowie seine Salze, Solvate und Solvate der Salze.

Weiterer Gegenstand der Erfindung ist ein Verfahren zur Herstellung der erfindungsgemäßen Verbindungen der Formel (I) dadurch gekennzeichnet, dass man eine Verbindung der Formel (II) 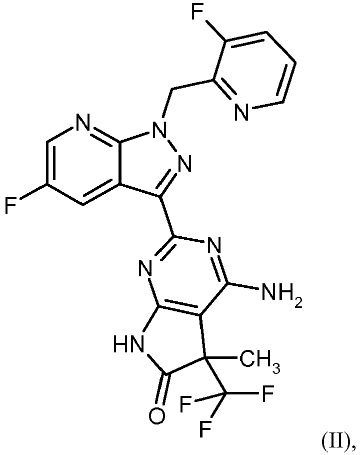 in einem inerten Lösungsmittel mit iso-Pentylnitrit und einem Halogen-Äquivalent in eine Verbindung der Formel (III) 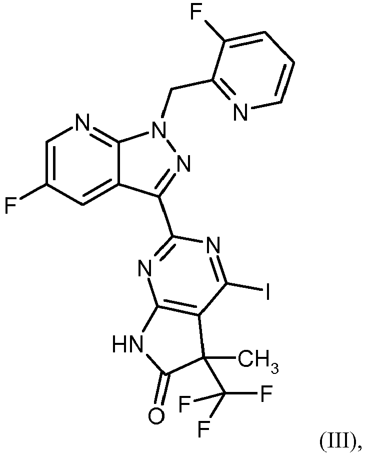 überführt, und diese im Anschluss in einem inerten Lösungsmittel gegebenenfalls in Gegenwart einer geeigneten Base mit einer Verbindung der Formel (IV) zu der Verbindung der Formel (I) 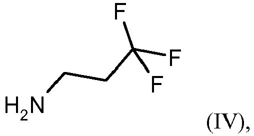 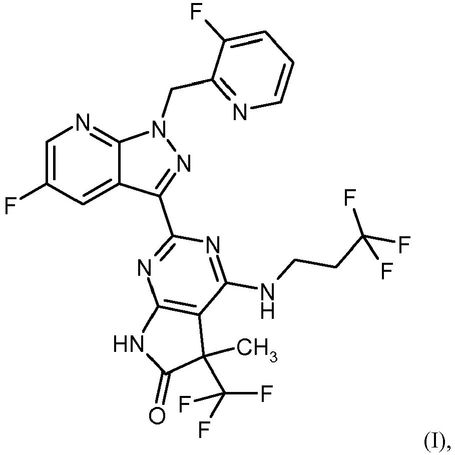 umsetzt,
und gegebenenfalls die resultierende Verbindung der Formel (I) gegebenenfalls mit den entsprechenden (i) Lösungsmitteln und/oder (ii) Säuren oder Basen in ihre Solvate, Salze und/oder Solvate der Salze überführt.

Der Verfahrensschritt (II) → (III) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dioxan.

Die Reaktion (II) → (III) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Halogen-Quelle bei der Umsetzung (II) → (III) eignen sich beispielsweise Diiodmethan, eine Mischung aus Cäsiumiodid, Iod und Kupfer-(I)-iodid oder Kupfer-(II)-bromid.

Inerte Lösungsmittel für den Verfahrensschritt (III) + (IV) → (I) sind beispielsweise Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'*-Dimethylpropylenharnstoff (DMPU), *N*-Methylpyrrolidon (NMP), Pyridin, Acetonitril oder Sulfolan. Ebenso ist es möglich, Gemische der genannten Lösungsmittel einzusetzen. Bevorzugt ist NMP.

Die Reaktion (III) + (IV) → (I) wird im Allgemeinen in einem Temperaturbereich von +20°C bis +200°C, bevorzugt bei +150°C bis +200°C, bevorzugt in einer Mikrowelle, durchgeführt. Die Umsetzung kann bei normalem, erhöhtem oder bei erniedrigtem Druck erfolgen (z.B. von 0.5 bis 5 bar).

Das beschriebene Herstellverfahren kann durch das folgende Syntheseschema (Schema 1) verdeutlicht werden: 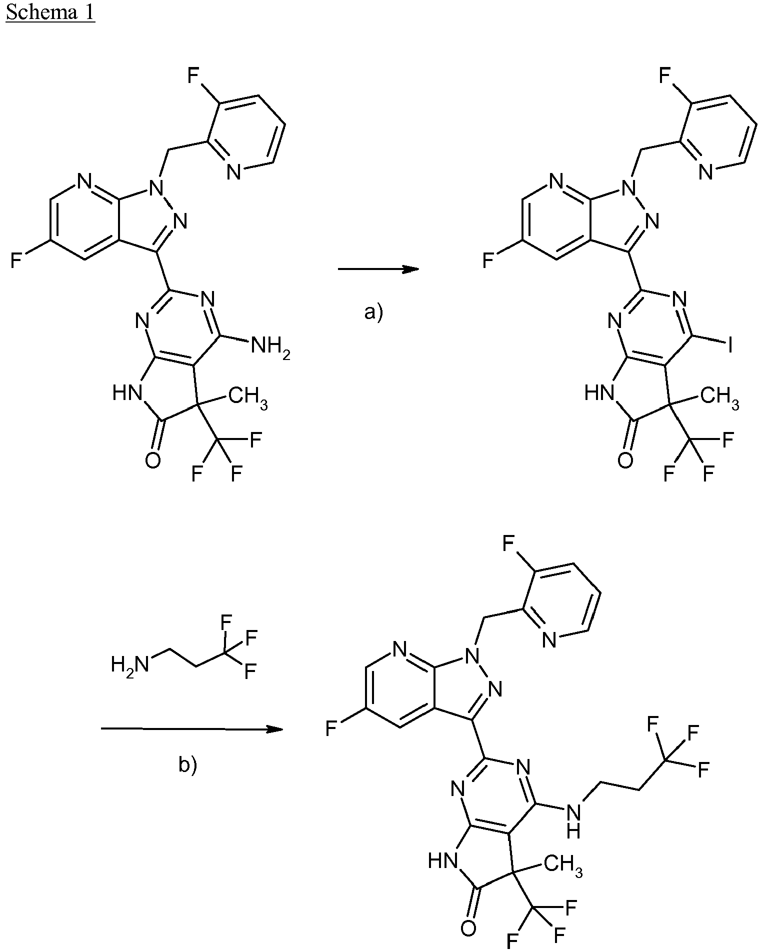 [a): Diiodmethan, iso-Pentylnitrit; b): NMP, Mikrowelle, 150°C].

Die Verbindungen der Formel (II) können hergestellt werden, indem man eine Verbindung der Formel (V) 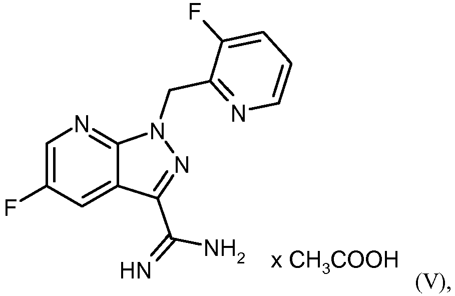 in einem inerten Lösungsmittel in Gegenwart einer geeigneten Base mit einer Verbindung der Formel (VI) 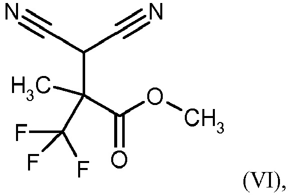 zu der Verbindung der Formel (II) 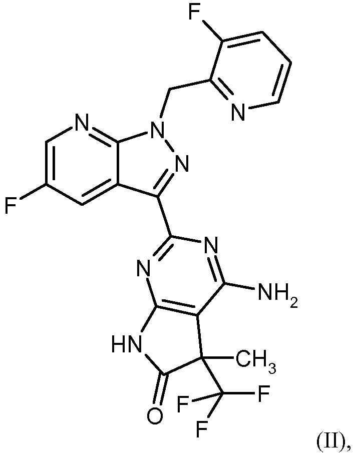 umsetzt.

Inerte Lösungsmittel für den Verfahrensschritt (V) + (VI) → (II) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist tert.-Butanol.

Geeignete Basen für den Verfahrensschritt (V) + (VI) → (II) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktion (V) + (VI) → (II) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Das zuvor beschriebene Herstellverfahren kann durch das nachfolgende Syntheseschema (Schema 3) beispielhaft verdeutlicht werden: 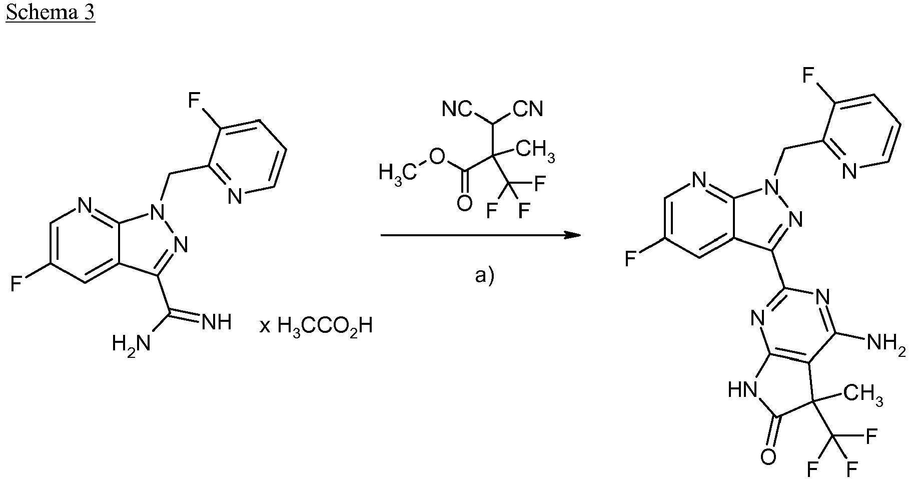 [a): KOt-Bu, tert.-Butanol].

Die Verbindungen der Formel (V) sind literaturbekannt (siehe z.B. WO 2013/004785, Beispiel 14A).

Die Verbindung der Formel (VI) kann hergestellt werden, indem man eine Verbindung der Formel (VII) 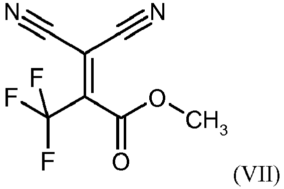 in einem inerten Lösungsmittel mit Methylmagnesiumhalogenid umsetzt.

Die Verbindung der Formel (VII) ist literaturbekannt (vgl. z.B. Journal of Fluorine Chemistry, 1991, vol. 51, # 3 S. 323 - 334.).

Die Verbindung der Formel (IV) ist kommerziell erhältlich, literaturbekannt oder kann in Analogie zu literaturbekannten Verfahren hergestellt werden.

Die erfindungsgemäßen Verbindungen wirken als potente Stimulatoren der löslichen Guanylatcyclase, besitzen wertvolle pharmakologische Eigenschaften, und weisen ein verbessertes therapeutisches Profil auf, wie beispielsweise hinsichtlich ihrer *in-vivo* Eigenschaften und/oder ihres pharmakokinetischen Verhaltens und/oder metabolischen Profils. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärkt die erfindungsgemäße Verbindung die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus kann die erfindungsgemäße Verbindung auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzhei-mer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Desweiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Offenbarung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Offenbarung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Offenbarung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Offenbarung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), Edoxaban (DU-176b), Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- DCI: direkte chemische Ionisation (bei MS)
- DMF: Dimethylformamid
- DMSO: Dimethylsulfoxid
- d. Th.: der Theorie (bei Ausbeute)
- ESI: Elektrospray-Ionisation (bei MS)
- h: Stunde(n)
- HPLC: Hochdruck-, Hochleistungsflüssigchromatographie
- LC/MS: Flüssigchromatographie-gekoppelte Massenspektrometrie
- Min: Minute(n)
- MS: Massenspektrometrie
- NMR: Kernresonanzspektrometrie
- RT: Raumtemperatur
- Rt: Retentionszeit (bei HPLC)
- t-Bu: tert.-Butyl
- TFA: Trifluoressigsäure
- THF: Tetrahydrofuran
- v/v: Volumen zu Volumen-Verhältnis (einer Lösung)

### HPLC- und LC/MS-Methoden:

### Methode 1 (LC-MS):

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen und Intermediate:

### Beispiel 1A

### 5-Fluor-1-[(3-fluorpyridin-2-yl)methyl]-1H-pyrazolo[3,4-b]pyridin-3-carboximidamid Acetat

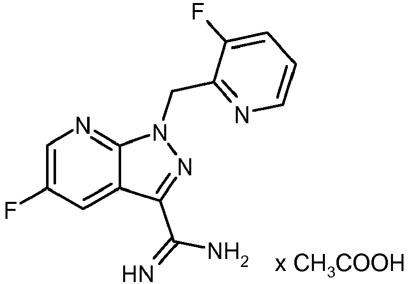

Die Herstellung der Verbindung ist beschrieben in WO2013/004785, Beispiel 14A, S. 69-70.

### Beispiel 2A

### Methyl-3,3-dicyan-2-(trifluormethyl)acrylat

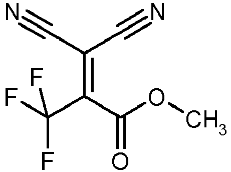

Die Synthese dieser Verbindung ist beschrieben in Journal of Fluorine Chemistry, 1991, vol. 51, # 3 S. 323 - 334.

### Beispiel 3A

### Methyl-2-(dicyanmethyl)-3,3,3-trifluor-2-methylpropanoat

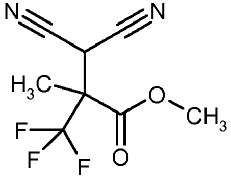

3.00 g (14.698 mmol) Beispiel 2A wurden in Tetrahydrofuran (30 ml) gelöst und auf 0°C abgekühlt. Anschliessend wurden 7.35 ml (22.047 mmol) Methylmagnesiumchlorid (3M in THF) so zugetropft, dass die Temperatur 5°C nicht überstieg. Nach vollständiger Zugabe wurde 10 min nachgerührt. Der Ansatz wurde dann mit IN wässriger Salzsäure versetzt und anschliessend mit Ethylacetat extrahiert. Die Phasen wurden getrennt und die wässrige Phase wurde noch zweimal mit Ethylacetat extrahiert. Die vereinigten organischen Phasen wurden mit gesättigter wässriger Natriumchlorid-Lösung gewaschen, über Natriumsulfat getrocknet, filtriert und eingeengt. Das Rohprodukt wurde anschliessend über Kieselgel chromatographiert (Laufmittel: Cyclohexan, danach Cyclohexan:Ethylacetat 9:1 (v:v). Nach Einengen erhielt man 3.24 g (63 % d. Th.) der Titelverbindung.
¹H-NMR (400 MHz, CDCl₃): δ [ppm] = 1.81 (s, 3H), 3.95 (s, 3H), 4.48 (s, 1H).

### Beispiel 4A

### 4-Amino-2-{5-fluor-1-[(3-fluorpyridin-2-yl)methyl]-1H-pyrazolo[3,4-b]pyridin-3-yl}-5-methyl-5-(trifluormethyl)-5,7-dihydro-6H-pyrrolo[2,3-d]pyrimidin-6-on

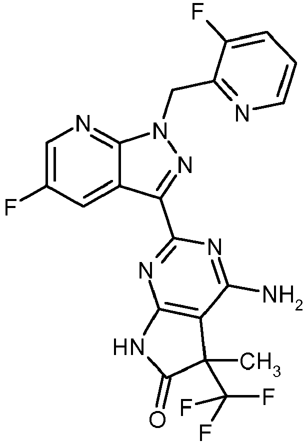

23.0 g (66.023 mmol) Beispiel 1A wurden in tert.-Butanol (400 ml) vorgelegt und mit 13.43 g (119.683 mmol) Kalium-tert.-butylat versetzt. Anschliessend wurden 21.079 g (95.746 mmol) Beispiel 3A in tert.-Butanol (100 ml) zugegeben und die Mischung über Nacht zum Rückfluss erhitzt. Nach Abkühlen wurde das Reaktionsgemisch mit Wasser versetzt und für 30 min bei Raumtemperatur gerührt. Der entstandene Niederschlag wurde abfiltriert und mit Wasser und wenig Diethlyether nachgewaschen. Der Feststoff wurde am Hochvakuum getrocknet. Es wurden 16.1 g der Titelverbindung erhalten (51% d. Th.).
LC-MS (Methode 1): Rₜ = 0.95 min; MS (ESIpos): m/z = 477 [M+H]⁺
¹H-NMR (400 MHz, DMSO-d₆): δ [ppm] = 1.72 (s, 3H), 5.96 (s, 2H), 7.10 (br s, 2H), 7.42-7.48 (m, 1H), 7.75-7.80 (m, 1H), 8.27 (d, 1H), 8.68 (dd, 1H), 8.86 (dd, 1H), 11.60 (br s, 1H).

### Beispiel 5A

### 2-{5-Fluor-1-[(3-fluorpyridin-2-yl)methyl]-1H-pyrazolo[3,4-b]pyridin-3-yl}-4-iod-5-methyl-5-(trifluormethyl)-5,7-dihydro-6H-pyrrolo[2,3-d]pyrimidin-6-on

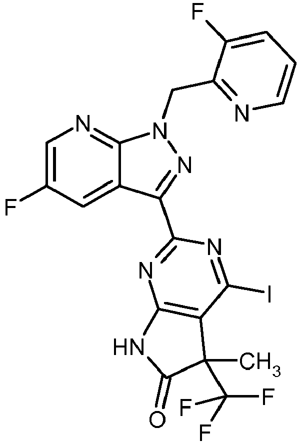

565 mg (1.186 mmol) von Beispiel 4A in Dioxan (15 ml) wurden mit 798 µl (5.930 mmol) iso-Pentylnitrit und 286 µl (3.558 mmol) Diiodmethan versetzt und für 4h auf 85°C erhitzt. Nach Abkühlen wurde im Vakuum eingeengt, der Rückstand mit Dichlormethan aufgenommen, mit Kieselgur versetzt und anschliessend im Vakuum eingeengt. Die so auf Kieselgur aufgezogene Rohverbindung wurde anschliessend über Kieselgel chromatographiert (Laufmittel: Cyclohexan :Ethylacetat Gradient). Nach Einengen erhielt man 297 mg (42 % d. Th.) der Titelverbindung.
LC-MS (Methode 1): Rₜ = 1.19 min; MS (ESIpos): m/z = 588 [M+H]⁺
¹H-NMR (400 MHz, DMSO-d₆): δ [ppm] = 1.81 (s, 3H), 6.04 (s, 2H), 7.43-7.47 (m, 1H), 7.77-7.82 (m, 1H), 8.26 (d, 1H), 8.47 (dd, 1H), 8.76 (dd, 1H), 12.41 (br s, 1H).

### Ausführungsbeispiele:

### Beispiel 1

### 2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on

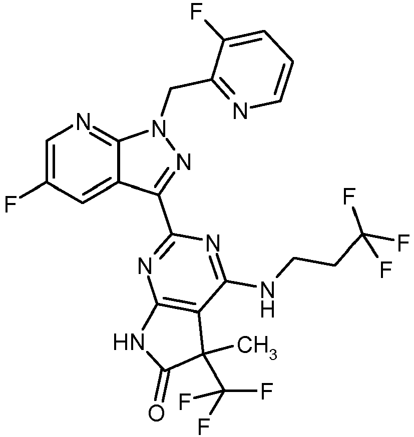

293 mg (0.462 mmol, ca. 92% Reinheit) Beispiel 5A wurden in einem mikrowellengeeigneten Reaktionsgefäß in 1-Methyl-2-pyrrolidon (4.5 ml) gelöst und mit 126 µl (1.409 mmol) 3,3,3-Trifluorpropyl-1-amin versetzt. Anschliessend wurde mit einem Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 168 mg der Titelverbindung erhalten (62 % d. Th., Reinheit 97 %).
LC-MS (Methode 1): Rₜ = 1.15 min; MS (EIpos): m/z = 573 [M+H]⁺.
¹H-NMR (400 MHz, DMSO-d₆): δ [ppm] = 1.73 (s, 3H), 2.64-2.71 (m, 2H), 3.79-3.93 (m, 2H), 5.99 (s, 2H), 6.91 (t, 1H), 7.42-7.46 (m, 1H), 7.78 (t, 1H), 8.28 (d, 1H), 8.46 (dd, 1H), 8.71 (s br, 1H), 11.71 (br s, 1H).

### Trennung in Enantiomere:

301 mg Racemat (vereinigte Menge Beispiel 1 aus 2 unterschiedlichen Ansätzen) wurden mittels präparativer HPLC (Laufmittel: (iso-Hexan/Ethanol 30/70 (v/v), Fluss 15 ml/min, Temperatur 40°C, Wellenlänge: 220 nm) an chiraler Phase (Daicel Chiralpak AZ-H, 5 µM, 250 x 20 mm) in die Enantiomere aufgetrennt.

### Beispiel 1-1 (Enantiomer 1)

### (5S)-2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on

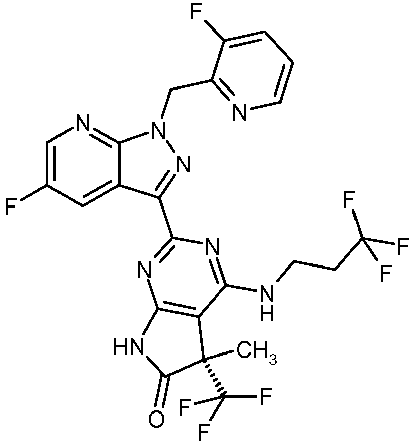

Ausbeute: 138 mg
Rt = 4.056 min; ee > 99% [analytische HPLC, Laufmittel: iso-Hexan/Ethanol 30/70 (v/v), Fluss 1 ml/min, Temperatur 40°C, Wellenlänge: 220 nm, an chiraler Phase (Daicel Chiralpak AZ-H, 5 µM 250 x 4.6 mm)].

Eine Einkristall-Röntgenstrukturanalyse bestätigte für dieses Enantiomer die S-Konfiguration.

### Beispiel 1-2 (Enantiomer 2)

### (5R)-2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on

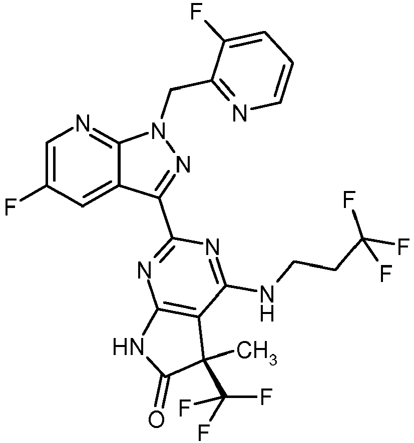

Ausbeute: 145 mg
Rt = 5.576 min; ee > 99% [analytische HPLC, Laufmittel: (iso-Hexan/Ethanol 30/70 (v/v), Fluss 1 ml/min, Temperatur 40°C, Wellenlänge: 220 nm; an chiraler Phase (Daicel Chiralpak AZ-H, 5 µM, 250 x 4.6 mm].

### B. Bewertung der pharmakolosischen Wirksamkeit

Im Folgenden werden die nachstehenden Abkürzungen verwendet:
- BSA: Bovines Serumalbumin
- EDTA: Ethylendiamintetraessigsäure
- µCi: Micro Curie
- Tris: Tris(hydroxymethyl)-aminomethan

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 1-1 | 265 |
| 1-2 | 237 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| **Beispiel** | **MEC [µM]** |
|---|---|
| 1-1 | 0.1 |
| 1-2 | 1.0 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Einleitung der Narkose Gas O₂ + N₂O 30:50 mit 5% Isofluran in einem Durchflussbehälter.

Nach Einleitung der Narkose wird das Tier auf einer Wärmeplatte an die Narkosemaske angeschlossen und erhält zur Narkoseerhaltung 1,8% Isofluran.narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum (Oxytetracyclin® 10%, 60 mg/kg s.c., 0.06 ml/100g body weight, Beta-Pharma GmbH & Co, Germany) sowie ein Analgetikum (Rimadyl®, 4 mg/kg s.c., Pfizer, Germany) verabreicht

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 2 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (30 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden nach Nummern sortiert in Ordnern abgelegt.

Repräsentative Werte für die erfindungsgemäßen Verbindungen sind in der nachstehenden Tabelle (Tabelle 3) wiedergegeben:

**Tabelle 3:**

| **Beispiel 1-1** | | | |
|---|---|---|---|
| | **Vehikel 2ml/kg** | **Dosis: 0.3 mg/kg p.o.** | **Dosis: 0.03mg/kg mg/kg** |
| Stunden nach Substanzgabe | mittlerer Blutdruck (mmHg) | mittlerer Blutdruck (mmHg) | mittlerer Blutdruck (mmHg) |
| 0 | 143,6 | 138,7 | 148,8 |
| 1 | 142,0 | 116,3 | 133,5 |
| 2 | 138,5 | 106,8 | 127,3 |
| 3 | 141,2 | 107,2 | 128,8 |
| 4 | 141,5 | 105,5 | 132,3 |
| 5 | 138,7 | 106,2 | 126,0 |
| 6 | 136,7 | 106,8 | 124,5 |
| 7 | 147,8 | 101,7 | 138,8 |
| 8 | 154,7 | 111,3 | 143,8 |
| 9 | 143,3 | 109,0 | 136,5 |
| 10 | 150,8 | 113,3 | 143,8 |
| 11 | 149,2 | 114,8 | 141,7 |
| 12 | 150,7 | 112,0 | 141,7 |
| 13 | 151,7 | 117,2 | 150,5 |
| 14 | 148,8 | 115,7 | 150,8 |
| 15 | 155,3 | 117,8 | 153,8 |
| 16 | 150,3 | 111,5 | 145,7 |
| 17 | 155,5 | 121,7 | 155,3 |
| 18 | 156,7 | 132,3 | 151,2 |
| 19 | 160,2 | 117,0 | 146,3 |
| 20 | 144,5 | 110,7 | 140,2 |
| 21 | 151,5 | 124,0 | 142,2 |
| 22 | 145,5 | 125,5 | 142,8 |
| 23 | 165,0 | 139,0 | 143,0 |
| 24 | 171,5 | 150,5 | 143,2 |

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen der Formel (I) werden in männlichen CD-1-Mäusen, männlichen Wister-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen der Formel (I), Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer PufferLösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC (Area Under the Curve), Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000 g wird die Konzentration im Plasma gemessen (mittels LC-MS/MS; s.o.) und durch Quotientenbildung der C_{Blut}/Cₚₗₐₛₘₐ-Wert ermittelt.

Tabelle 4 zeigt Daten repräsentativer Verbindungen der vorliegenden Erfindung nach intravenöser Gabe von 0.3 mg/kg sowie peroraler Gabe von 1 mg/kg in Ratten:

**Tabelle 4:**

| Beispiel | 1-1 |
|---|---|
| AUCₙₒᵣₘ [kg·h/L] | 1.77 |
| CL_{Blut} [L/h/kg] | 0.70 |
| MRT [h] | 6.6 |
| t_{1/2} [h] | 5.9 |
| F [%] | 98.8 |

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-6. Inhibition der bovinen Phosphodiesterase 6 (PDE 6)

PDE 6 wird aus Photorezeptor-Außensegmenten von Rinder-Retina (ROS) gereinigt, durch milde Trypsinierung aktiviert und durch Ionenaustauscher-Chromatographie mittels einer Mono Q Säule weiter gereinigt. Fraktionen die PDE 6 Aktivität aufweisen werden vereinigt (PDE 6-Präparat) und bei -80° gelagert. (I. Saenz de Tejada et al., International Journal of Impotence Research 2001, 13, 282-290). Die in vitro-Wirkung von Testsubstanzen auf bovine PDE 6 wird mit dem kommerziell verfügbaren 3H-cGMP Scintillation Proximity Assay (SPA) von Perkin Elmer bestimmt. Die Reaktion (20 min, 37°C) enthält serielle Verdünnungsreihen der Testsubstanzen in DMSO sowie PDE 6-Präparat (typische Verdünnung 1:100000), [8-³H] guanosine 3', 5'-cyclic phosphate (0,25 µCi/mL; Perin Elmer) und unmarkiertes cGMP (10 µM) in Assaypuffer (50 mM Tris/HCl pH 7,5; 140 mM NaCl; 8,3 mM MgCl₂; 1,7 mM EDTA; 0,02% BSA). IC₅₀-Werte werden anhand der graphischen Auftragung der Substanzkonzentration gegen die prozentuale PDE 6-Inhibition bestimmt (Wunder et al. Molecular Pharmacology 2005, 68, 1775-1781).

Repräsentative IC₅₀-Werte für die erfindungsgemäßen Verbindungen sind in der nachstehenden Tabelle (Tabelle 5) wiedergegeben:

**Tabelle 5:**

| Beispiel Nr. | IC₅₀ [nM] |
|---|---|
| 1-1 | 220 |
| 1-2 | 1400 |

### B-7. Bestimmung der Organ-protektiven Wirkungen im Langzeitversuch an Ratten.

Die Organ-protektiven Wirkungen der sGC Stimulatoren wurden in einem therapeutisch relevanten "low nitric oxide (NO) / high renin" Hypertoniemodell an der Ratten gezeigt. Die Studie wurde in Anlehnung an die kürzlich erschienene Publikation durchgeführt (Sharkovska Y, Kalk P, Lawrenz B, Godes M, Hoffmann LS, Wellkisch K, Geschka S, Relle K, Hocher B, Stasch JP. NOindependent stimulation of soluble guanylate cyclase reduces target organ damage in lowand high-renin models of hypertension. J. Hypertension. 2010; 28: 1666-1675). Dabei wurden Renin-transgene Ratten (TGR(mRen2)27), denen der NO-Synthase-Inhibitor L-NAME über das Trinkwasser verabreicht wurde, gleichzeitig mit einem sGC Stimualtor oder Vehikel über mehrere Wochen behandelt. Haemodynamische und renale Parameter wurden während des Behanlungszeitraums bestimmt. Am Ende der Langzeitstudie wurde die Organprotektion (Niere, Lunge, Herz, Aorta) durch histopathologische Untersuchungen, Biomarker, Expressionsanalysen und kardiovaskuläre Plasmaparameter gezeigt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung mit dem systematischen Namen 2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)-amino]-5,7-dihydro-6H-pyrrolo[2,3-d]pyrimidin-6-on und der Strukturformel (I) 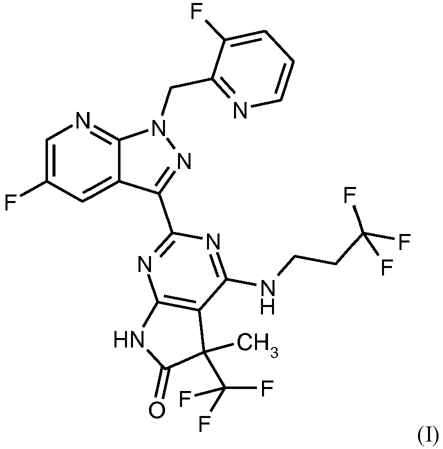 sowie ihre Salze, Solvate und Solvate der Salze.

2. Enantiomer der Verbindung (I) mit dem systematischen Namen (5R)-2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on und der Strukturformel (I-A) 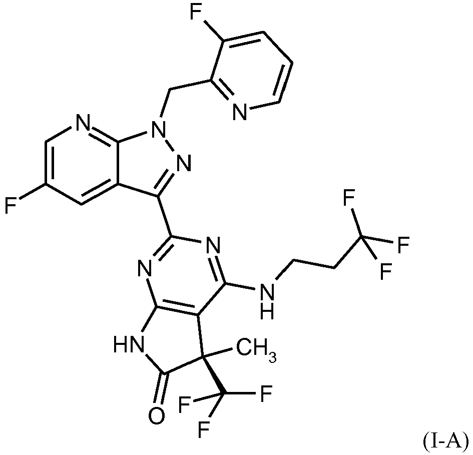 sowie seine Salze, Solvate und Solvate der Salze.

3. Enantiomer der Verbindung (I) mit dem systematischen Namen (5S)-2-{5-Fluor-1-[(3-fluorpyridin-2-yl)methyl]-1H-pyrazolo[3,4-b]pyridin-3-yl}-5-methyl-5-(trifluormethyl)-4-[(3,3,3-trifluorpropyl)amino]-5,7-dihydro-6H-pyrrolo[2,3-d]pyrimidin-6-on und der Strukturformel (I-B) 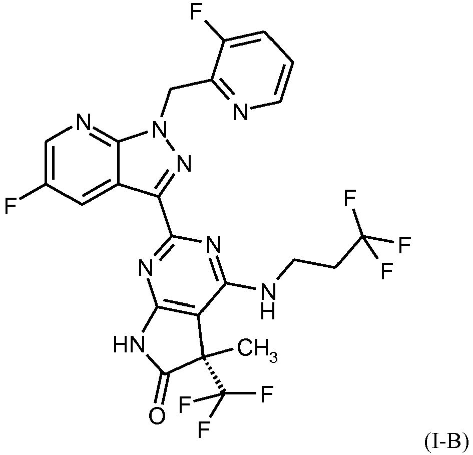 sowie seine Salze, Solvate und Solvate der Salze.

4. Verfahren zur Herstellung von Verbindung der Formel (I), wie in den Anspruch 1 definiert, **dadurch gekennzeichnet, dass** man eine Verbindung der Formel (II) 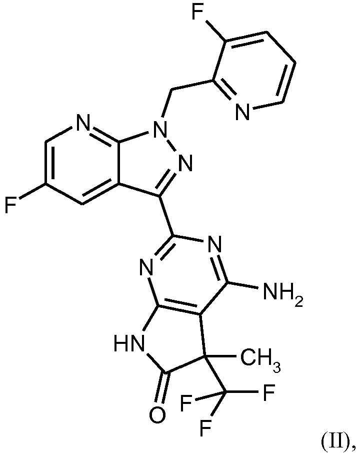 in einem inerten Lösungsmittel mit iso-Pentylnitrit und einem Halogen-Äquivalent in eine Verbindung der Formel (III) 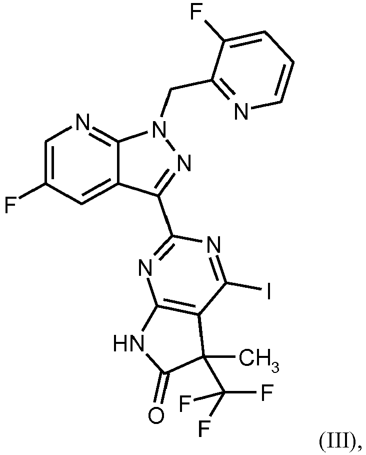 überführt, und diese im Anschluss in einem inerten Lösungsmittel gegebenenfalls in Gegenwart einer geeigneten Base mit einer Verbindung der Formel (IV) 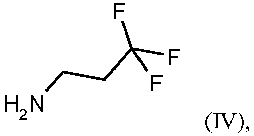 zu der Verbindung der Formel (I) 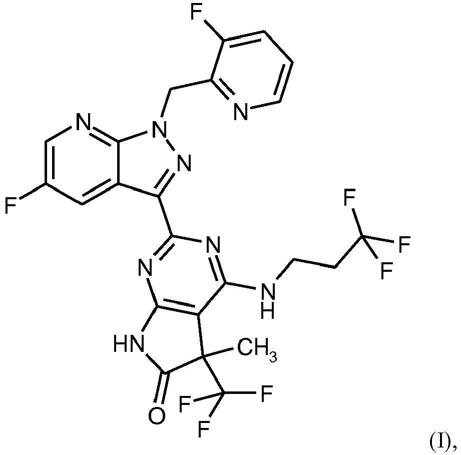 umsetzt, und gegebenenfalls die resultierende Verbindung der Formel (I) gegebenenfalls mit den entsprechenden (i) Lösungsmitteln und/oder (ii) Säuren oder Basen in ihre Solvate, Salze und/oder Solvate der Salze überführt.

5. Verfahren gemäß Anspruch 4, **dadurch gekennzeichnet, dass** die Verbindung der Formel (I) in ihre Enantiomere getrennt wird.

6. Verbindung der Formel (I), (I-A) oder (I-B), wie in den Ansprüchen 1, 2 oder 3 definiert, zur Verwendung in einem Verfahren zur Behandlung und/oder Prophylaxe von Krankheiten.

7. Verwendung einer Verbindung der Formel (I), (I-A) oder (I-B), wie in den Ansprüchen 1, 2 oder 3 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

8. Verbindung der Formel (I), (I-A) oder (I-B), wie in den Ansprüchen 1, 2 oder 3 definiert, zur Verwendung in einem Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

9. Arzneimittel enthaltend eine Verbindung der Formel (I), (I-A) oder (I-B), wie in den Ansprüchen 1, 2 oder 3 definiert, in Kombination mit einem inerten, nicht-toxischen, pharmazeutisch geeigneten Hilfsstoff.

10. Arzneimittel enthaltend eine Verbindung der Formel (I), (I-A) oder (I-B), wie in den Ansprüchen 1, 2 oder 3 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

11. Arzneimittel nach Anspruch 9 oder 10 zur Verwendung in einem Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound having the systematic name 2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one and the structural formula (I) 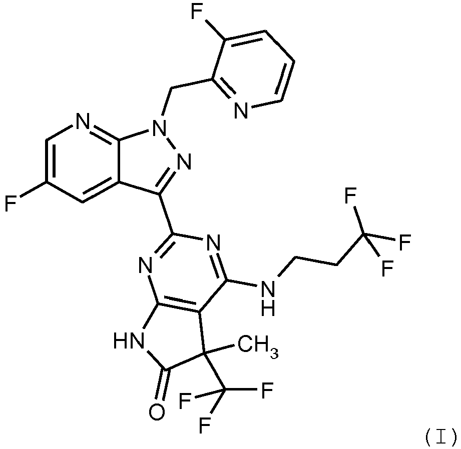 and the salts, solvates and solvates of the salts thereof.

2. Enantiomer of the compound (I) having the systematic name (5R)-2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one and the structural formula (I-A) 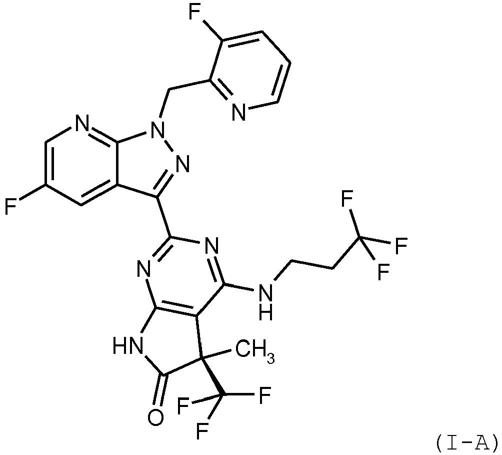 and the salts, solvates and solvates of the salts thereof.

3. Enantiomer of the compound (I) having the systematic name (5S)-2-{5-fluoro-1-[(3-fluoropyridin-2-yl)methyl]-1H-pyrazolo[3,4-b]pyridin-3-yl}-5-methyl-5-(trifluoromethyl)-4-[(3,3,3-trifluoropropyl)amino]-5,7-dihydro-6H-pyrrolo[2,3-d]pyrimidin-6-one and the structural formula (I-B) 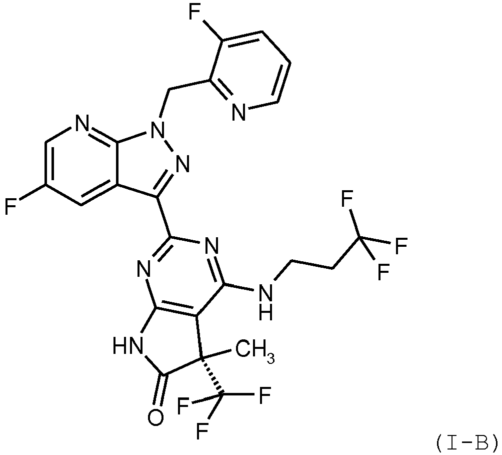 and the salts, solvates and solvates of the salts thereof.

4. Process for preparing compounds of the formula (I) as defined in Claim 1, **characterized in that** a compound of the formula (II) 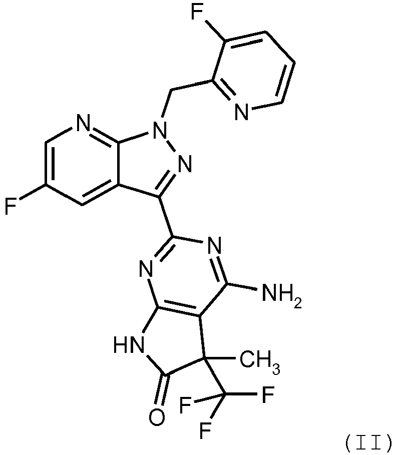 is converted in an inert solvent using isopentyl nitrite and a halogen equivalent into a compound of the formula (III) 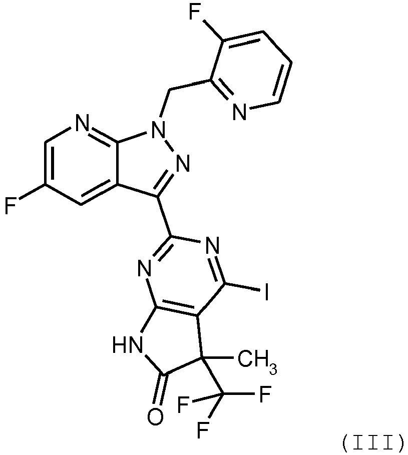 and this is then reacted in an inert solvent, optionally in the presence of a suitable base, with a compound of the formula (IV) 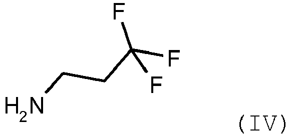 to give a compound of the formula (I) 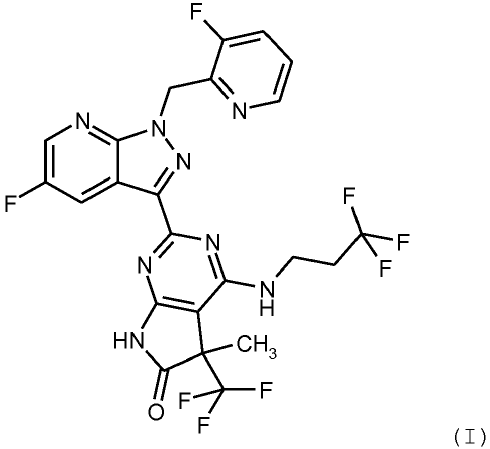 and the resulting compound of the formula (I) is optionally converted with the appropriate (i) solvents and/or (ii) acids or bases to the solvates, salts and/or solvates of the salts thereof.

5. Process according to Claim 4, **characterized in that** the compound of formula (I) is separated into its enantiomers.

6. Compound of the formula (I), (I-A) or (I-B) as defined in Claim 1, 2 or 3 for use in a method for the treatment and/or prophylaxis of diseases.

7. Use of a compound of the formula (I), (I-A) or (I-B) as defined in Claim 1, 2 or 3 for producing a medicament for the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

8. Compound of the formula (I), (I-A) or (I-B) as defined in Claim 1, 2 or 3 for use in a method for the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

9. Medicament comprising a compound of the formula (I), (I-A) or (I-B) as defined in Claim 1, 2 or 3 in combination with an inert, non-toxic, pharmaceutically suitable auxiliary.

10. Medicament comprising a compound of the formula (I), (I-A) or (I-B) as defined in Claim 1, 2 or 3 in combination with a further active compound selected from the group consisting of organic nitrates, NO donors, cGMP-PDE inhibitors, agents having antithrombotic activity, agents lowering blood pressure, and agents altering lipid metabolism.

11. Medicament according to Claim 9 or 10 for use in a method for the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

## Revendications

1. Composé ayant le nom systématique 2-{5-fluoro-1-[(3-fluoropyridin-2-yl)méthyl]-1H-pyrazolo[3,4-b]pyridin-3-yl}-5-méthyl-5-(trifluorométhyl)-4-[(3,3,3-trifluoropropyl)-amino]-5,7-dihydro-6H-pyrrolo[2,3-d]pyrimidin-6-one et la formule structurale (I) 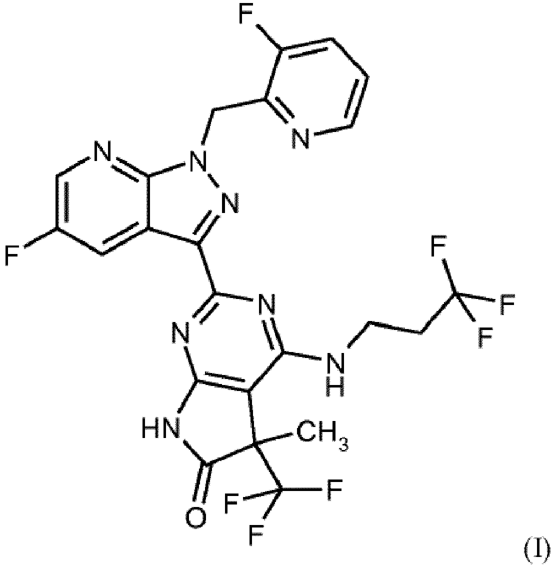 ainsi que ses sels, solvates et solvates des sels.

2. Énantiomère du composé (I) ayant le nom systématique (5R)-2-{5-fluoro-1-[(3-fluoropyridin-2-yl)méthyl]-1H-pyrazolo[3,4-b]pyridin-3-yl}-5-méthyl-5-(trifluorométhyl)-4-[(3,3,3-trifluoropropyl)-amino]-5,7-dihydro-6H-pyrrolo[2,3-d]pyrimidin-6-one et la formule structurale (I-A) 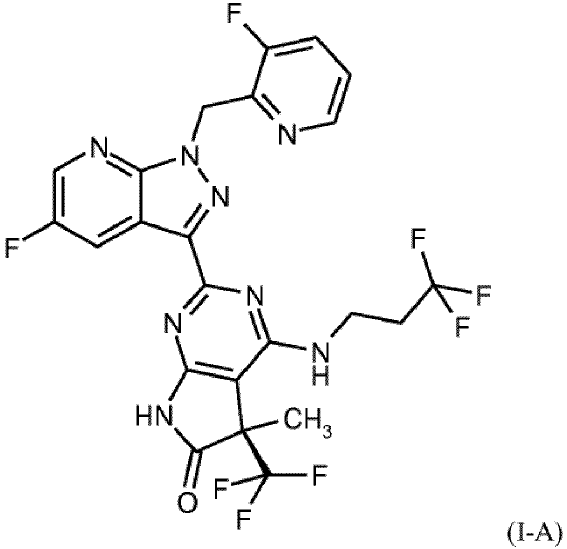 ainsi que ses sels, solvates et solvates des sels.

3. Énantiomère du composé (I) ayant le nom systématique (5S)-2-{5-fluoro-1-[(3-fluoropyridin-2-yl)méthyl]-1H-pyrazolo[3,4-b]pyridin-3-yl}-5-méthyl-5-(trifluorométhyl)-4-[(3,3,3-trifluoropropyl)-amino]-5,7-dihydro-6H-pyrrolo[2,3-d]pyrimidin-6-one et la formule structurale (I-B) 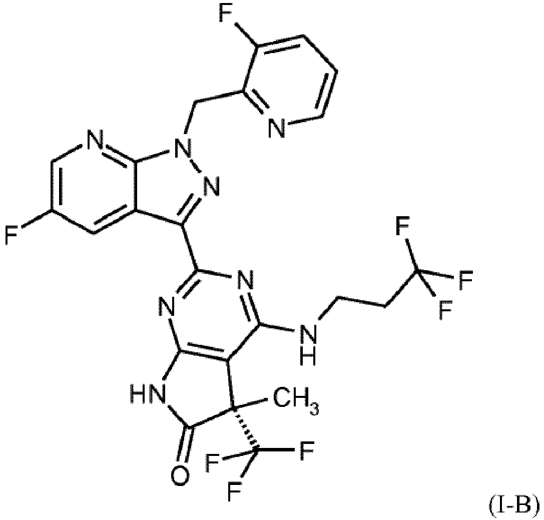 ainsi que ses sels, solvates et solvates des sels.

4. Procédé de fabrication du composé de formule (I), tel que défini dans la revendication 1, **caractérisé en ce qu'**un composé de formule (II) 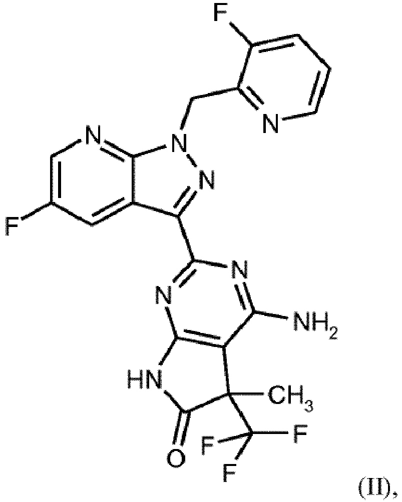 est transformé dans un solvant inerte avec de l'iso-pentylnitrite et un équivalent d'halogène en un composé de formule (III) 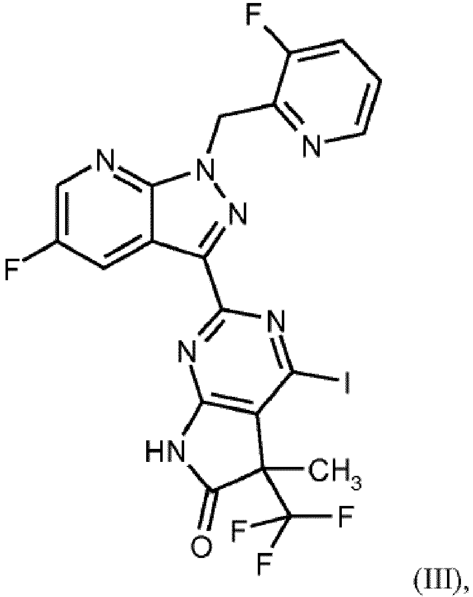 puis celui-ci est mis en réaction dans un solvant inerte éventuellement en présence d'une base appropriée avec un composé de formule (IV) 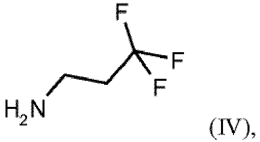 pour former le composé de formule (I) 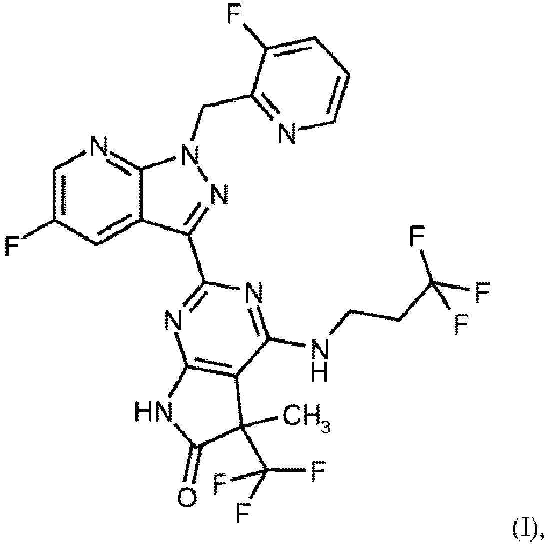 et le composé de formule (I) résultant est éventuellement transformé avec (i) les solvants et/ou (ii) les acides ou les bases appropriés en ses solvates, sels et/ou solvates des sels.

5. Procédé selon la revendication 4, **caractérisé en ce que** le composé de formule (I) est séparé en ses énantiomères.

6. Composé de formule (I), (I-A) ou (I-B), tel que défini dans les revendications 1, 2 ou 3, destiné à une utilisation dans un procédé de traitement et/ou de prophylaxie de maladies.

7. Utilisation d'un composé de formule (I), (I-A) ou (I-B), tel que défini dans les revendications 1, 2 ou 3, pour la fabrication d'un médicament pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.

8. Composé de formule (I), (I-A) ou (I-B), tel que défini dans les revendications 1, 2 ou 3, destiné à une utilisation dans un procédé de traitement et/ou de prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.

9. Médicament, contenant un composé de formule (I), (I-A) ou (I-B), tel que défini dans les revendications 1, 2 ou 3, en combinaison avec un adjuvant inerte, non toxique, pharmaceutiquement approprié.

10. Médicament, contenant un composé de formule (I), (I-A) ou (I-B), tel que défini dans les revendications 1, 2 ou 3, en combinaison avec un autre agent actif choisi dans le groupe constitué par les nitrates organiques, les donneurs de NO, les inhibiteurs de cGMP-PDE, les agents à effet antithrombotique, les agents abaissant la tension artérielle, ainsi que les agents modifiant le métabolisme lipidique.

11. Médicament selon la revendication 9 ou 10, destiné à une utilisation dans un procédé de traitement et/ou de prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.
